# EUROPEAN PATENT SPECIFICATION

(11) **EP 3 710 012 B1**
(45) Date of publication and mention of the grant of the patent: **11.06.2025**
(21) Application number: 19746564.4
(22) Date of filing: 01.02.2019
(51) Int. Cl.: A61K 31/568, A61K 9/00, A61P 5/26, A61P 9/00, A61P 15/08

(54) **METHODS OF TESTOSTERONE THERAPY**
VERFAHREN FÜR TESTOSTERON-THERAPIE
PROCÉDÉS DE THÉRAPIE À LA TESTOSTÉRONE

(30) Priority: 02.02.2018 US 201862625653 P; 07.11.2018 US 201862756976 P
(43) Date of publication of application: 23.09.2020
(73) Proprietor: Acerus Biopharma Inc., Mississauga, ON L5L 1J9 (CA)
(72) Inventor: WESTFIELD, Gerwin, Dublin, Ohio 43016 (US); ZWIERKO, Margaux, Huntersville, North Carolina 28078 (US); RAMASAMY, Ranjith, Pinecrest, Florida 33156 (US); BRYSON, Nathan, Toronto, Ontario M4V 1Y9 (CA)
(74) Representative: Reddie & Grose LLP
(86) International application number: PCT/US2019/016373
(87) International publication number: WO 2019/152854

(56) References cited:
- WO-A1-2012/156820
- WO-A1-2012/156820
- CN-A- 105 362 667
- US-A1- 2006 222 708
- US-A1- 2011 034 544
- US-A1- 2013 143 851
- US-A1- 2013 143 851
- RAMASAMY RANJITH ET AL: "Effect of Natesto on Reproductive Hormones, Semen Parameters and Hypogonadal Symptoms: A Single Center, Open Label, Single Arm Trial", JOURNAL OF UROLOGY, vol. 204, no. 3, 1 September 2020 (2020-09-01), BALTIMORE, MD, US, pages 557 - 563, XP055814114, ISSN: 0022-5347, Retrieved from the Internet <URL:https://www.auajournals.org/doi/pdf/10.1097/JU.0000000000001078> DOI: 10.1097/JU.0000000000001078
- ROY ET AL.: "Association of Testosterone Levels With Anemia in Older Men A Controlled Clinical Trial", JAMA INTERNAL MEDICINE, vol. 177, 21 February 2017 (2017-02-21), pages 480 - 490, XP055706646
- MAIA ET AL.: "Pulsatile administration of testosterone by the vaginal route using Pentravan", MONDUZZI EDITORIALE, 2013, pages 181 - 184, XP055629163

## Description

### CROSS-REFERENCE TO RELATED APPLICATIONS

This application claims priority under 35 U.S.C. § 119(e) to U.S. Provisional Patent Application No. 62/625,653, filed on February 2, 2018; and U.S. Provisional Patent Application No. 62/756,976, filed November 7, 2018.

### FIELD OF THE INVENTION

The present disclosure is generally directed to testosterone replacement therapy (TRT), and particularly to testosterone for use in methods for preventing or reducing side effects of TRT by administering a testosterone formulation multiple times per day.

### BACKGROUND OF THE INVENTION

Testosterone is an anabolic steroid and the primary male sex hormone, promoting development of male reproductive tissues such as the prostate and testes. It can activate androgen receptors in its unchanged form, or it can be converted to 5α-dihydrotestosterone (DHT) by the enzyme 5α-reductase before binding to the androgen receptor. Once bound, the receptor-hormone complex moves into the cell nucleus, altering specific gene sequences on the cellular DNA and modifying its transcription to promote the synthesis of proteins that form testosterone-sensitive tissues.

One of the several male reproductive processes that rely on testosterone is spermatogenesis, in which primitive germ cells known as spermatogonia divide to produce spermatocytes, which form young sperm cells known as spermatids, which mature into fully grown sperm cells known as spermatozoa. Adequate testosterone levels are especially crucial in the final spermatid maturation step and to ensure normal semen quality (i.e. a sperm count of at least 20 million spermatozoa per milliliter semen).

Low testosterone, also known as low T or hypogonadism, is a condition in which the testes produce insufficient testosterone and is defined as a serum total testosterone level of less than 300 nanograms per deciliter (ng/dL). Low testosterone affects more than 10% of men worldwide, with higher incidence in the elderly, the chronically ill, and those with various other modifiable risk factors, such as obesity and diabetes. Testosterone deficiencies are associated with a wide range of other negative health outcomes in men, including lower muscle mass, bone mineral density, and hematocrit and hemoglobin concentrations; smaller prostate glands; and diminished energy and sexual function relative to men with normal testosterone levels. Particularly, low testosterone levels can result in oligozoospermia (lower than normal sperm count in semen) or even azoospermia (no sperm in semen).

In recent years, testosterone replacement therapy (TRT), in which a pharmaceutical composition containing testosterone, or a salt, ester, or prodrug thereof, is administered by any of several known means, has become a standard treatment for low testosterone. Current delivery systems for TRT include transdermal gels and patches, injectable compositions, and long-acting subcutaneous pellets.

While TRT has been shown to increase serum testosterone levels to normal ranges and improve patients' bone mineral density, prostate volume, energy, and sexual function, the therapy can suppress pituitary gonadotropins, especially follicle-stimulating hormone (FSH) and luteinizing hormone (LH), which can result in lower testicular volume and a corresponding impairment of spermatogenesis, semen quality (i.e. oligozoospermia or azoospermia), and therefore fertility. Various drugs, including clomiphene citrate, anastrozole, and human chorionic gonadotropin (HCG), have sometimes been administered to counteract these effects, but these drugs also have various undesirable side effects, including decreased libido and gynecomastia.

Moreover, the safety of TRT remains a primary concern. Most TRT delivery modes, especially injection of testosterone esters, can increase patients' hemoglobin and hematocrit concentrations, which can result in a condition known as secondary polycythemia. Unmanaged, polycythemia can lead to such complications as jaundice, pruritus, cerebrovascular accident, thrombosis, and bleeding. Additionally, elevated hematocrit levels are associated with an increased risk of death from cardiovascular disease.

Due to these and other drawbacks, treatment options for men suffering from low testosterone that are both safe and effective remain limited, especially for men already at risk for one or more of the negative side effects of TRT. There is thus a significant and long-felt need in the art for methods of treating low testosterone that increase serum testosterone levels and patients' sperm counts to normal levels, while mitigating or eliminating the unsafe and/or undesirable effects resulting from FSH and LH suppression and elevated hemoglobin and hematocrit levels.

US2013/0143851A1 describes therapeutic methods for providing an increase in serum testosterone levels and methods for treating a disease or a symptom associated with deficient endogenous levels of testosterone.

WO2012/156820A1 describes pernasal testosterone bio-adhesive gel formulations for providing sustained intranasal delivery of testosterone to testosterone deficient males.

CN105362667A describes a traditional Chinese medicine extract for prevention and treatment of spermatogenesis dysfunction.

### SUMMARY OF THE INVENTION

The present invention is as defined in the appended claims.

In particular, in one aspect of the present invention there is provided testosterone for use in a method of treating a patient at risk of, or in need of avoiding, at least one side effect associated with testosterone replacement therapy (TRT) or a pituitary gonadotropin deficiency, the method comprising pulsatile administration of testosterone to the patient, wherein the side effect is selected from the group consisting of azoospermia, oligozoospermia, decreased libido, gynecomastia, cardiovascular disease, and cardiovascular accident, and wherein pulsatile administration comprises administering multiple doses to the nasal mucosae of the patient, with each dose administered no less than three hours and no more than 24 hours after an immediately preceding dose.

In another aspect of the present invention, there is provided testosterone for use in a method of treating a sexual disorder in a male human, the method comprising pulsatile administration of testosterone to the male human, wherein the sexual disorder is selected from the group consisting of azoospermia, oligozoospermia, decreased libido, and gynecomastia, and wherein pulsatile administration comprises administering multiple doses to the nasal mucosae of the patient, with each dose administered no less than three hours and no more than 24 hours after an immediately preceding dose.

The testosterone may, but need not, be administered to the nasal mucosae of the patient in the form of a testosterone gel.

In embodiments, the testosterone may be administered to the patient in at least two doses per day. The testosterone may, but need not, be administered to the patient in at least three doses per day. The testosterone may, but need not, be administered to the patient in at least four doses per day.

In embodiments, pulsatile administration may comprise administering multiple doses and each dose comprises between about 5 mg and about 15 mg testosterone. Each dose may, but need not, comprise about 11 mg testosterone.

In embodiments, a total amount of testosterone administered to the patient per day may be between about 10 mg and about 120 mg. The total amount of testosterone administered to the patient per day may, but need not, be between about 20 mg and about 40 mg.

In embodiments, the method may not comprise administration of any drug selected from the group consisting of clomiphene citrate, anastrozole, and human chorionic gonadotropin (HCG).

After at least about two weeks of treatment, at least one of the following may be true: (i) a level of follicle-stimulating hormone (FSH) in the patient is between about 1.5 IU/L and about 12.4 IU/L; (ii) a level of luteinizing hormone (LH) in the patient is at least about 1.80 IU/L; (iii) a hematocrit of the patient is less than about 60%; and (iv) a level of hemoglobin in the patient is less than about 20.0 g/dL.

The advantages of the present invention will be apparent from the disclosure contained herein.

### BRIEF DESCRIPTION OF THE DRAWINGS

Figure 1 is an illustration of a study design used to obtain the experimental results of Example 1.
Figure 2 is a graph of the mean follicle-stimulating hormone (FSH) and luteinizing hormone (LH) concentrations of the subjects of the study of Example 1 who received an intranasal testosterone formulation twice daily (BID), at the start of the study and at 90 days.
Figure 3 is a graph of the mean serum testosterone levels of the subjects of the study of Example 1 who received an intranasal testosterone formulation BID on day 90.
Figure 4 is a graph of the mean follicle-stimulating hormone (FSH) and luteinizing hormone (LH) concentrations of the subjects of the study of Example 1 who received an intranasal testosterone formulation three times daily (TID), at the start of the study and at 90 days.
Figure 5 is a graph of the mean serum testosterone levels of the subjects of the study of Example 1 who received an intranasal testosterone formulation TID on day 90.

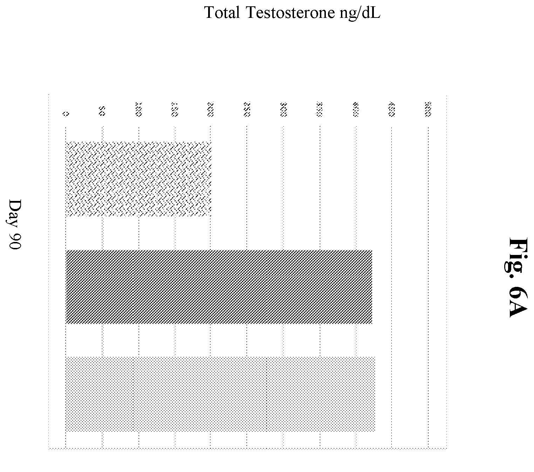

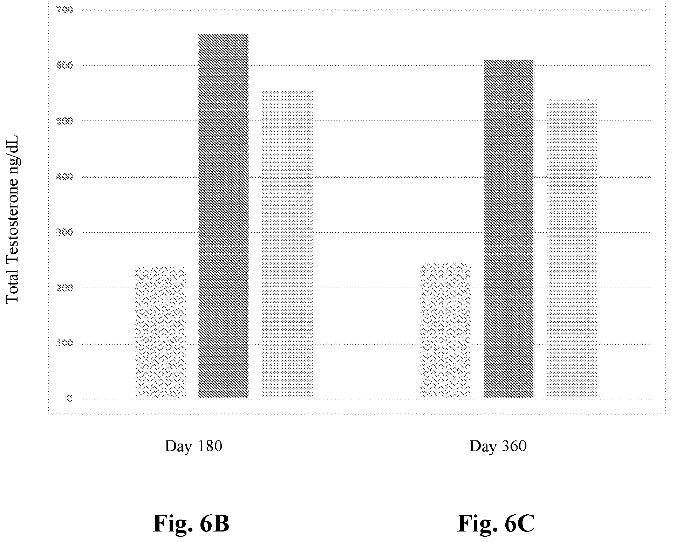

Figures 6A, 6B, and 6C are graphs of the mean serum testosterone levels of the subjects of the study of Example 1, relative to a pre-study baseline, at 90, 180, and 360 days, respectively.
Figures 7A, 7B, and 7C are graphs of the mean serum testosterone levels, FSH and LH concentrations, and semen parameters, respectively, of the subjects of the study of Example 2.

### DETAILED DESCRIPTION OF THE INVENTION

As used herein, the phrases "at least one," "one or more," "or," and "and/or" are openended expressions that are both conjunctive and disjunctive in operation. For example, each of the expressions "at least one of A, B and C," "at least one of A, B, or C," "one or more of A, B, and C," "one or more of A, B, or C," "A, B, and/or C," and "A, B, or C" means A alone, B alone, C alone, A and B together, A and C together, B and C together, or A, B, and C together.

As used herein, the term "pulsatile administration" and related terms refer to methods of administration of a drug to a patient that result in a pulsatile dosing profile.

As used herein, the term "pulsatile dosing profile" and related terms refer to dosing profiles characterized by alternating periods of relatively low drug concentrations in the patient and relatively high drug concentrations in the patient.

Embodiments of the present invention relate to testosterone for use in a method of treating a patient at risk of, or in need of avoiding, at least one side effect associated with testosterone replacement therapy (TRT) or a pituitary gonadotropin deficiency, the method comprising pulsatile administration of testosterone to the patient, wherein the side effect is selected from the group consisting of azoospermia, oligozoospermia, decreased libido, gynecomastia, cardiovascular disease, and cardiovascular accident, and wherein pulsatile administration comprises administering multiple doses to the nasal mucosae of the patient, with each dose administered no less than three hours and no more than 24 hours after an immediately preceding dose.

Embodiments of the present invention further relate to testosterone for use in a method of treating a sexual disorder in a male human, the method comprising pulsatile administration of testosterone to the male human, wherein the sexual disorder is selected from the group consisting of azoospermia, oligozoospermia, decreased libido, and gynecomastia, and wherein pulsatile administration comprises administering multiple doses to the nasal mucosae of the patient, with each dose administered no less than three hours and no more than 24 hours after an immediately preceding dose.

The present invention reduces known or suspected risks associated with TRT, and as a result, men who may be at risk, or wish to mitigate the risk, of azoospermia, oligozoospermia, decreased libido, gynecomastia, cardiovascular disease, or other side effects associated with conventional TRT methods can receive the benefits of TRT. By way of non-limiting example, men who wish to mitigate the risk of azoospermia, oligozoospermia, and/or decreased libido may include men who wish to attempt to conceive, and who therefore require normal semen quality and, thus, fertility.

Pulsatile dosing regimens according to embodiments of the present invention may result in periods during which a serum testosterone level of a patient is less than about 600 ng/dL, less than about 550 ng/dL, less than about 500 ng/dL, less than about 450 ng/dL, less than about 400 ng/dL, less than about 350 ng/dL, less than about 300 ng/dL, less than about 250 ng/dL, less than about 200 ng/dL, less than about 150 ng/dL, less than about 100 ng/dL, or less than about 50 ng/dL, or alternatively any whole number value less than about 600 ng/dL. Pulsatile dosing regimens according to embodiments of the present invention may also result in periods during which a serum testosterone level of a patient is in a normal range, e.g. more than about 300 ng/dL, more than about 350 ng/dL, more than about 400 ng/dL, more than about 450 ng/dL, more than about 500 ng/dL, more than about 550 ng/dL, more than about 600 ng/dL, more than about 650 ng/dL, more than about 700 ng/dL, more than about 750 ng/dL, or more than about 800 ng/dL, or alternatively any whole number value more than about 300 ng/dL.

Pulsatile dosing regimens according to embodiments of the present invention may result in a cycle between a low serum testosterone level in a patient and a high testosterone level in the patient; by way of non-limiting example, this cycle may recur once per day, twice per day, three times per day, four times per day, five times per day, six times per day, seven times per day, eight times per day, or more than eight times per day, i.e. the patient may exhibit a high and/or low serum testosterone level once, twice, three times, four times, five times, six times, seven times, eight times, or more than eight times per day. Stated another way, the cycle between a low serum testosterone level in the patient and a high serum testosterone level in the patient may have a period of less than about 24 hours, less than about twelve hours, less than about eight hours, less than about six hours, less than about 4.8 hours, less than about four hours, less than about 3.4 hours, or less than about three hours.

Pulsatile dosing regimens according to the present invention are administered to the patient by intranasal pulsatile administration, i.e. pulsatile administration to the nasal mucosae. A preferred formulation for delivery of testosterone in this embodiment is NATESTO^{®}, a form of TRT that is delivered intranasally to men suffering from low testosterone and that avoids the side effects common to other TRT delivery methods. In addition to the mitigation or elimination of adverse side effects, advantages of the use of NATESTO^{®} in conjunction with the present invention include ease of delivery; lack of needles; and especially decreased risk of accidental dosing of persons other than the patient, i.e. women or children, to which transdermal systems are particularly susceptible.

In embodiments of the present invention, a testosterone formulation in the form of a gel may be administered to a patient in need thereof by pulsatile administration to the nasal mucosae of the patient. The gel may be administered solely to the nasal mucosae within a left nostril of the patient, solely to the nasal mucosae within a right nostril of the patient, or to the nasal mucosae within both left and right nostrils of the patient.

In embodiments of the present invention, a testosterone formulation in a form other than a gel may be administered to a patient in need thereof by pulsatile administration to the nasal mucosae of the patient. By way of non-limiting example, testosterone formulations in these embodiments may take the form of a solution, a suspension, a cream, an ointment, a paste, and/or a powder.

In embodiments of the present invention, a testosterone formulation may be administered to a patient in need thereof once per day, twice per day, three times per day, four times per day, five times per day, six times per day, seven times per day, eight times per day, or more than eight times per day. In preferred embodiments, the testosterone formulation is administered to the patient twice per day or three times per day.

In embodiments of the present invention, a patient in need of TRT may receive between about 1 mg and about 31 mg of testosterone per dose, more preferably between about 2 mg and about 29 mg of testosterone per dose, more preferably between about 3 mg and about 27 mg of testosterone per dose, more preferably between about 4 mg and about 25 mg of testosterone per dose, more preferably between about 5 mg and about 23 mg of testosterone per dose, more preferably between about 6 mg and about 21 mg of testosterone per dose, more preferably between about 7 mg and about 19 mg of testosterone per dose, more preferably between about 8 mg and about 17 mg of testosterone per dose, more preferably between about 9 mg and about 15 mg of testosterone per dose, more preferably between about 10 mg and about 13 mg of testosterone per dose, and most preferably about 11 mg of testosterone per dose. In additional embodiments of the present invention, a patient in need of TRT may receive a total daily dose of testosterone between about 2 mg and about 53 mg, more preferably between about 7 mg and about 48 mg, more preferably between about 12 mg and about 43 mg, more preferably between about 17 mg and about 38 mg, and most preferably between about 22 mg and about 33 mg. In pulsatile dosing regimens according to the present invention, a total daily dose can be distributed, equally or unequally, between two or more individual pulsatile doses per day; likewise, an individual dose can be administered in a single step or can be split, equally or unequally, into two or more sub-doses, such as, by way of non-limiting example, into two equal sub-doses for administration to each of a left nostril and a right nostril of a patient.

In embodiments of the present invention, a patient suffering from low testosterone receives TRT but does not receive any drug commonly used to ameliorate the side effects of TRT, such as clomiphene citrate, anastrozole, or human chorionic gonadotropin (HCG), while nonetheless benefiting from a reduction or elimination of certain TRT side effects. This benefit is possible due to the effect of dosing regimens of the present invention on pituitary gonadotropins, especially follicle-stimulating hormone (FSH) and luteinizing hormone (LH).

The present inventors have surprisingly and unexpectedly found that embodiments of the present invention, in which a patient receives pulsatile administration of testosterone at least twice per day, maintain normal or near-normal levels of FSH and LH in the patient. Without intending to be bound by any particular theory, it is believed that embodiments of the present invention achieve this benefit by causing pulsatile release of gonadotropin-releasing hormone (GnRH), which prevents any sustained decrease in FSH and/or LH, due to the short half-life of the testosterone in the body. This benefit stands in contrast to conventional TRT methods, including and especially topical and transdermal delivery methods, which are known to suppress pituitary gonadotropins, including FSH and LH. Maintenance of normal or near-normal levels of FSH and LH in the patient represents a crucial advantage of the present invention relative to conventional TRT options at least because normal levels of FSH and LH allow a patient to avoid azoospermia and/or oligozoospermia during TRT, thus preserving normal sperm count, semen quality, and fertility, as may be desired, by way of non-limiting example, by men who wish to attempt to conceive during TRT.

In embodiments of the invention, a level of LH of a patient receiving testosterone by pulsatile administration is maintained at about 1.80 IU/L or more for at least about two weeks, at least about one month, at least about two months, at least about three months, at least about four months, at least about five months, at least about six months, at least about seven months, at least about eight months, at least about nine months, at least about ten months, at least about eleven months, and/or at least about one year. Moreover, in embodiments of the invention, a level of FSH of a patient receiving testosterone by pulsatile administration is maintained at between about 1.5 and about 12.4 IU/L for at least about two weeks, at least about one month, at least about two months, at least about three months, at least about four months, at least about five months, at least about six months, at least about seven months, at least about eight months, at least about nine months, at least about ten months, at least about eleven months, and/or at least about one year.

In embodiments of the present invention, a sperm count of a patient receiving testosterone by pulsatile administration is at least about 5 million spermatozoa per mL semen, at least about 10 million spermatozoa per mL semen, at least about 15 million spermatozoa per mL semen, at least about 20 million spermatozoa per mL semen, at least about 25 million spermatozoa per mL semen, at least about 30 million spermatozoa per mL semen, at least about 35 million spermatozoa per mL semen, at least about 40 million spermatozoa per mL semen, at least about 45 million spermatozoa per mL semen, or at least about 50 million spermatozoa per mL semen. In further embodiments, these sperm counts are maintained for at least about two weeks, at least about one month, at least about two months, at least about three months (or about 90 days), at least about four months, at least about five months, at least about six months (or about 180 days), at least about seven months, at least about eight months, at least about nine months (or about 270 days), at least about ten months, at least about eleven months, or at least about twelve months (or about 360 days).

The present inventors have surprisingly and unexpectedly found that embodiments of the present invention maintain a patient's hematocrit and hemoglobin levels within normal and/or acceptable ranges. Thus, another advantage of the present invention is in its suitability for use in patients having one or more cardiovascular risk factors, including but not limited to low physical activity level, tobacco use, poor diet, high blood lipid content, hypertension, obesity, family history, diabetes, age, ethnicity, and socioeconomic status. This benefit stands in contrast to conventional TRT methods, which are known to have an erythropoietic stimulating effect that can cause polycythemia, which may manifest as an increase in any one or more of hemoglobin, hematocrit, and red blood cell count. In embodiments, the hematocrit of a patient receiving TRT according to the present invention may be less than about 60%, less than about 55%, less than about 50%, less than about 45%, or less than about 40%, or alternatively any whole number percentage less than about 60%. Additionally or alternatively, a hemoglobin concentration in a patient receiving TRT according to the present invention may be less than about 20.0 g/dL, less than about 19.0 g/dL, less than about 18.0 g/dL, less than about 17.0 g/dL, less than about 16.0 g/dL, less than about 15.0 g/dL, less than about 14.0 g/dL, less than about 13.0 g/dL, or less than about 12.0 g/dL, or alternatively any tenth of a whole number value less than 20.0 g/dL. Such hematocrit and/or hemoglobin levels may be maintained for, by way of non-limiting example, at least about two weeks, at least about one month, at least about two months, at least about three months, at least about four months, at least about five months, at least about six months, at least about seven months, at least about eight months, at least about nine months, at least about ten months, at least about eleven months, or at least about one year.

The following experimental Examples serve to provide additional disclosure and illustration of the invention disclosed herein.

### Example 1

This Example illustrates that pulsatile administration of testosterone via a nasal testosterone gel (NTG), according to embodiments of the present invention, provides benefits related to pituitary gonadotropin levels not achievable with other exogenous testosterone preparations.

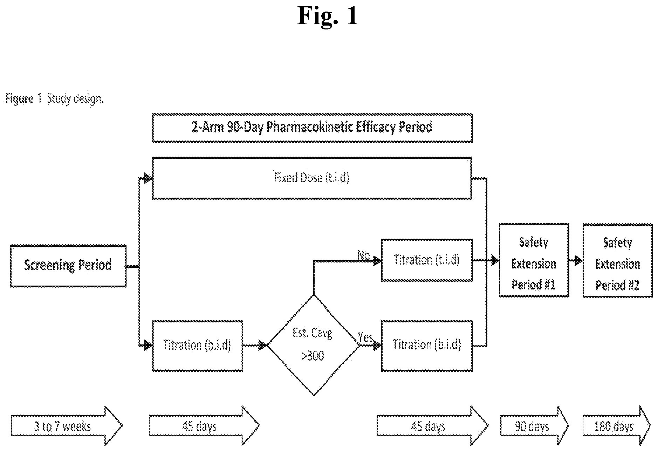

Men suffering from low testosterone were randomized into a 90-day open-label dose-ranging study. Each study subject self-administered a 4.5% NTG sold under the trade name NATESTO^{®} using a multiple-dose dispenser, either twice daily ("BID," n=122) or three times daily ("TID," n=151). Each dose comprised 11 mg testosterone, i.e. each subject received either 22 mg or 33 mg testosterone per day. Titration was performed based on blood levels to achieve a normal, or eugonadal, range of testosterone (300 to 1,050 ng/dL). Serum samples were obtained pre-study and after 90 days of treatment to determine relevant hormone levels, as shown in Figure 1. The mean subject characteristics of the study sample are characterized in Table 1 below.

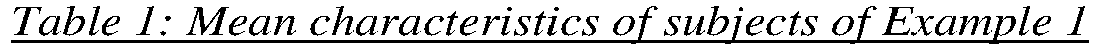

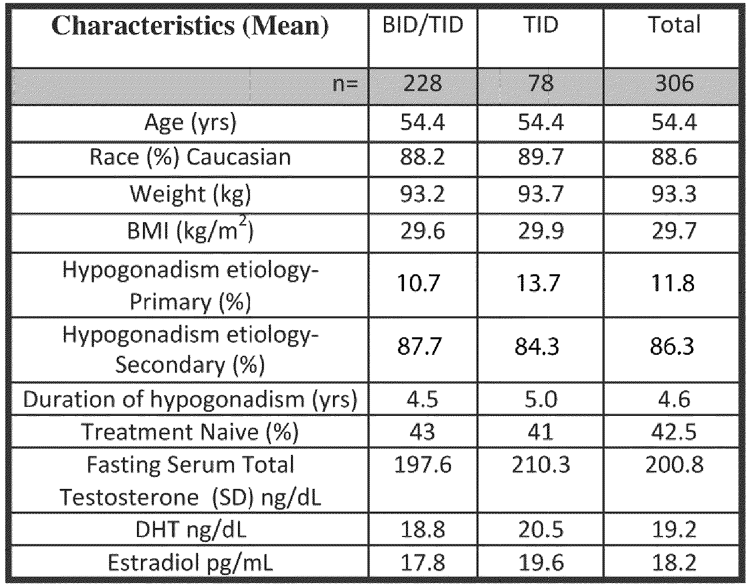

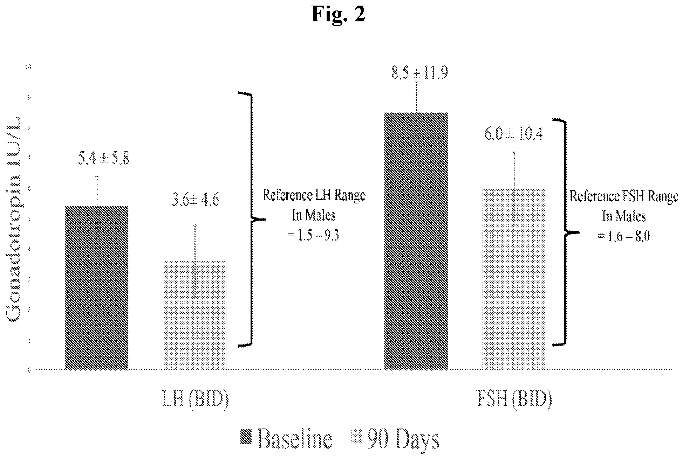

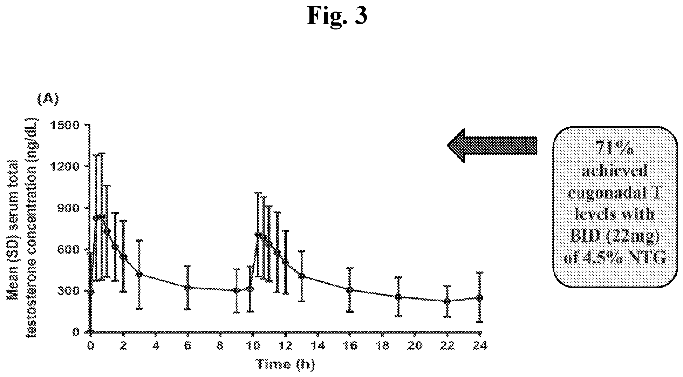

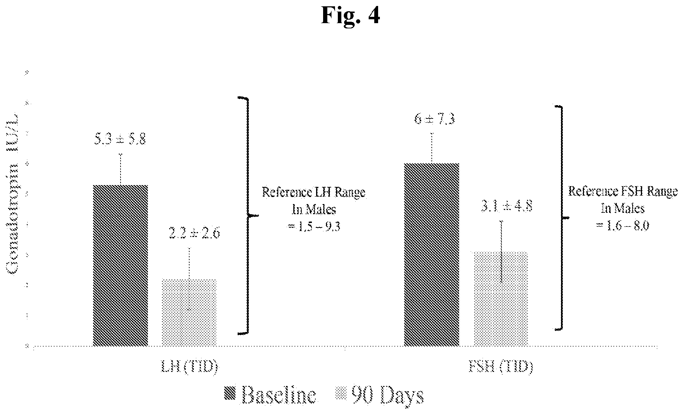

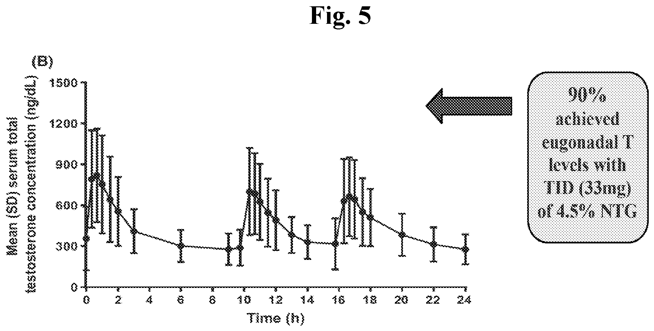

More detailed results are presented graphically in Figures 2 through 5. As shown in Table 1 and Figures 1 through 5, treatment with a 4.5% NTG generally restored serum total testosterone to eugonadal levels, and levels of pituitary gonadotropins were somewhat reduced but remained within standard and/or normal ranges for adult men. The data relating particularly to pituitary gonadotropins indicate that pulsatile administration of testosterone via an NTG, according to embodiments of the present invention, provides benefits not achievable with other exogenous testosterone preparations. Particularly, other exogenous testosterone preparations, especially those adapted for administration by injection, result in much greater suppression of pituitary gonadotropins. The present invention, by contrast, provides for a smaller decrease in FSH and LH levels in patients, and therefore may be suitable for patients who need or desire this benefit, e.g. men with naturally low FSH/LH levels or men who wish to attempt to conceive during TRT. The data further suggest that pulsatile administration of testosterone, at least twice per day, may have significant advantageous physiological effects relative to TRT regiments of the prior art, with the potential for positive physiological impact in testosterone-deficient patients. Novel treatment options, including but not limited to pulsatile administration of NATESTO^{®}, may have unique benefits in this regard.

In addition, hematocrit and hemoglobin values did not exceed the upper bound of the normal range in most patients tested in Example 1. After baseline screening, three subjects in the BID group (2.1%) and five subjects in the TID group (3.0%) had hematocrit and/or hemoglobin values above the upper bound of the normal range. No subject had a clinically significantly high hemoglobin or hematocrit value, and no subject had a post-treatment hematocrit value above 58%. Mean subject characteristics of the study sample are characterized in Table 2 below.

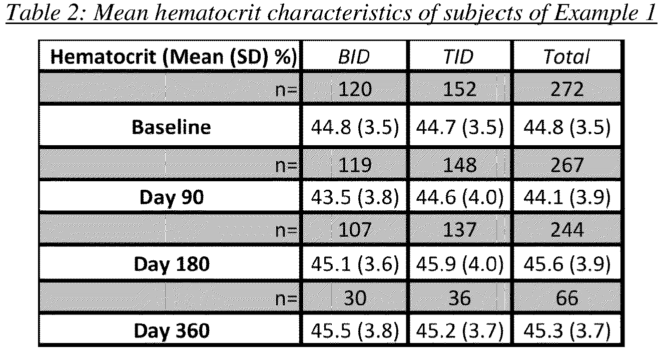

These data demonstrate that pulsatile administration, twice or more per day, of NTGs, including but not limited to NATESTO^{®}, allows men suffering from low testosterone to achieve normal serum total testosterone levels, while not increasing hematologic values to clinically significant values, and preferably not above normal values. The data thus allow the inference that pulsatile administration, twice or more per day, of NTGs, including but not limited to NATESTO^{®}, has a unique combination of safety and efficacy superior to that of conventional TRT regimens.

### Example 2

This Example illustrates that pulsatile administration of testosterone via a nasal testosterone gel (NTG), according to embodiments of the present invention, provides benefits related to sperm count not achievable with other exogenous testosterone preparations.

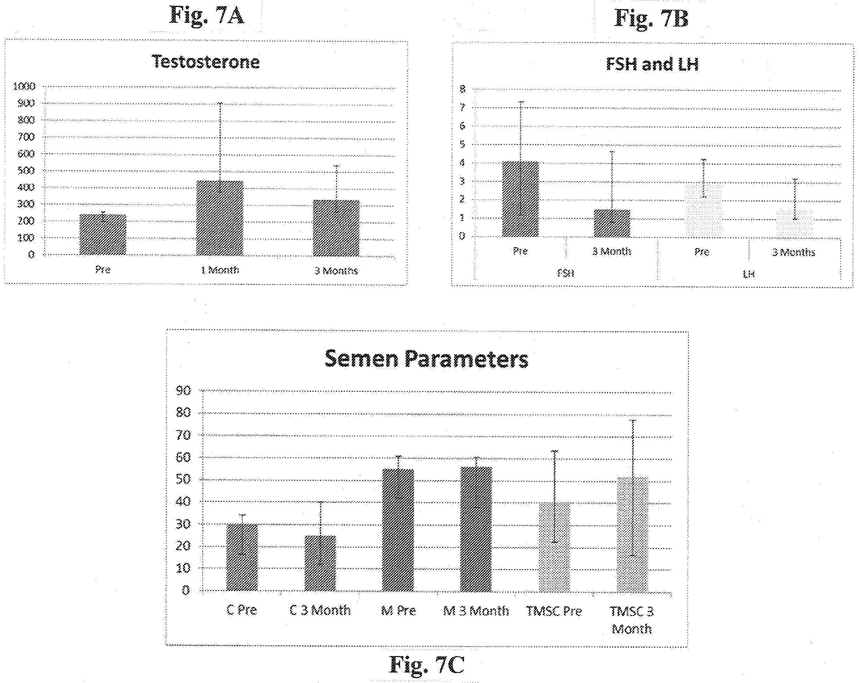

Baseline testosterone, FSH, LH, semen, IIEF-Q15, and SF-36 scores were obtained from six men aged 18-55, all of whom had serum total testosterone levels of less than 350 ng/dL and were naive to TRT prior to study. Each of the six men self-administered NATESTO^{®} (4.5% NTG) intranasally TID at 11 mg per dose (i.e. 33 mg per day). As shown in Figure 7A, after one month of therapy all six men had serum total testosterone levels of at least 379 ng/dL, with a median of 446.8 ng/dL, and after three months of therapy four of the six men had serum total testosterone levels of at least 300 ng/dL, with a median of 334.5 ng/dL. As shown in Figure 7B, after three months of the therapy, the six men also had a median LH level of 1.6 IU/L, and a median FSH level of 1.5 IU/L. Additionally, as shown in Figure 7C, the median total motile sperm count (TMSC) increased from 39.5 million at baseline to 52.0 million after three months of therapy.

## Claims

1. Testosterone for use in a method of treating a patient at risk of, or in need of avoiding, at least one side effect associated with testosterone replacement therapy (TRT) or a pituitary gonadotropin deficiency, the method comprising pulsatile administration of testosterone to the patient, wherein the side effect is selected from the group consisting of azoospermia, oligozoospermia, decreased libido, gynecomastia, cardiovascular disease, and cardiovascular accident, and wherein pulsatile administration comprises administering multiple doses to the nasal mucosae of the patient, with each dose administered no less than three hours and no more than 24 hours after an immediately preceding dose.

2. Testosterone for use in a method of treating a sexual disorder in a male human, the method comprising pulsatile administration of testosterone to the male human, wherein the sexual disorder is selected from the group consisting of azoospermia, oligozoospermia, decreased libido, and gynecomastia, and wherein pulsatile administration comprises administering multiple doses to the nasal mucosae of the patient, with each dose administered no less than three hours and no more than 24 hours after an immediately preceding dose.

3. Testosterone for use according to claim 1 or claim 2, wherein the testosterone is administered to the nasal mucosae of the patient in the form of a testosterone gel.

4. Testosterone for use according to claim 1 or claim 2, wherein the testosterone gel is administered to the patient in at least two doses per day.

5. Testosterone for use according to claim 4, wherein the testosterone gel is administered to the patient in at least three doses per day.

6. Testosterone for use according to 5, wherein the testosterone gel is administered to the patient in at least four doses per day.

7. Testosterone for use according to claim 1 or claim 2, wherein each dose comprises between about 5 mg and about 15 mg testosterone.

8. Testosterone for use according to claim 7, wherein each dose comprises about 11 mg testosterone.

9. Testosterone for use according to claim 1 or claim 2, wherein a total amount of testosterone administered to the patient per day is between about 10 mg and about 120 mg.

10. Testosterone for use according to claim 9, wherein the total amount of testosterone administered to the patient per day is between about 20 mg and about 40 mg.

11. Testosterone for use according to claim 1 or claim 2, not comprising administration of any drug selected from the group consisting of clomiphene citrate, anastrozole, and human chorionic gonadotropin (HCG).

## Patentansprüche

1. Testosteron zur Verwendung in einem Verfahren zur Behandlung eines Patienten, bei dem das Risiko von mindestens einer mit einer Testosteronersatztherapie (TRT) oder einem Hypophysen-Gonadotropin-Mangel assoziierten Nebenwirkung besteht oder der eine solche vermeiden muss, wobei das Verfahren das pulsatile Verabreichen von Testosteron an den Patienten umfasst, wobei die Nebenwirkung ausgewählt ist aus der Gruppe bestehend aus Azoospermie, Oligozoospermie, verminderter Libido, Gynäkomastie, Herz-Kreislauf-Erkrankung und Herz-Kreislauf-Unfall, und wobei die pulsatile Verabreichung das Verabreichen mehrerer Dosen an die Nasenschleimhäute des Patienten umfasst, wobei jede Dosis mindestens drei Stunden und höchstens 24 Stunden nach einer unmittelbar vorhergehenden Dosis verabreicht wird.

2. Testosteron zur Verwendung in einem Verfahren zur Behandlung einer sexuellen Störung bei einem männlichen Menschen, wobei das Verfahren das pulsatile Verabreichen von Testosteron an den männlichen Menschen umfasst, wobei die sexuelle Störung ausgewählt ist aus der Gruppe bestehend aus Azoospermie, Oligozoospermie, verminderter Libido und Gynäkomastie, und wobei die pulsatile Verabreichung das Verabreichen mehrerer Dosen an die Nasenschleimhäute des Patienten umfasst, wobei jede Dosis mindestens drei Stunden und höchstens 24 Stunden nach einer unmittelbar vorhergehenden Dosis verabreicht wird.

3. Testosteron zur Verwendung nach Anspruch 1 oder Anspruch 2, wobei das Testosteron in Form eines Testosterongels an die Nasenschleimhäute des Patienten verabreicht wird.

4. Testosteron zur Verwendung nach Anspruch 1 oder Anspruch 2, wobei das Testosterongel dem Patienten in mindestens zwei Dosen pro Tag verabreicht wird.

5. Testosteron zur Verwendung nach Anspruch 4, wobei das Testosterongel dem Patienten in mindestens drei Dosen pro Tag verabreicht wird.

6. Testosteron zur Verwendung nach Anspruch 5, wobei das Testosterongel dem Patienten in mindestens vier Dosen pro Tag verabreicht wird.

7. Testosteron zur Verwendung nach Anspruch 1 oder Anspruch 2, wobei jede Dosis zwischen etwa 5 mg und etwa 15 mg Testosteron umfasst.

8. Testosteron zur Verwendung nach Anspruch 7, wobei jede Dosis etwa 11 mg Testosteron umfasst.

9. Testosteron zur Verwendung nach Anspruch 1 oder Anspruch 2, wobei die Gesamtmenge an dem Patienten pro Tag verabreichtem Testosteron zwischen etwa 10 mg und etwa 120 mg liegt.

10. Testosteron zur Verwendung nach Anspruch 9, wobei die Gesamtmenge an dem Patienten pro Tag verabreichtem Testosteron zwischen etwa 20 mg und etwa 40 mg liegt.

11. Testosteron zur Verwendung nach Anspruch 1 oder Anspruch 2, die keine Verabreichung eines Arzneimittels aus der Gruppe bestehend aus Clomifencitrat, Anastrozol und humanem Choriongonadotropin (HCG) umfasst.

## Revendications

1. Testostérone pour utilisation dans une méthode de traitement d'un patient à risque, ou ayant besoin d'éviter, au moins un effet secondaire associé à une thérapie de remplacement de la testostérone (TRT) ou à un déficit en gonadotrophines hypophysaires, la méthode comprenant l'administration pulsatile de testostérone au patient, où l'effet secondaire est sélectionné parmi le groupe composé d'azoospermie, d'oligozoospermie, de diminution de la libido, de gynécomastie, de maladie cardiovasculaire et d'accident cardiovasculaire, et où l'administration pulsatile comprend administrer des doses multiples aux muqueuses nasales du patient, avec chaque dose étant administrée pas moins de trois heures et pas plus de 24 heures après une dose immédiatement précédente.

2. Testostérone pour utilisation dans une méthode de traitement d'un trouble sexuel chez un être humain mâle, la méthode comprenant l'administration pulsatile de testostérone à l'être humain mâle, où le trouble sexuel est sélectionné parmi le groupe composé d'azoospermie, d'oligozoospermie, de diminution de la libido et de gynécomastie, et où l'administration pulsatile comprend administrer des doses multiples aux muqueuses nasales du patient, avec chaque dose étant administrée pas moins de trois heures et pas plus de 24 heures après une dose immédiatement précédente.

3. Testostérone pour utilisation selon la revendication 1 ou la revendication 2, où la testostérone est administrée aux muqueuses nasales du patient sous forme d'un gel de testostérone.

4. Testostérone pour utilisation selon la revendication 1 ou la revendication 2, où le gel de testostérone est administré au patient en au moins deux doses par jour.

5. Testostérone pour utilisation selon la revendication 4, où le gel de testostérone est administré au patient en au moins trois doses par jour.

6. Testostérone pour utilisation selon la revendication 4, où le gel de testostérone est administré au patient en au moins quatre doses par jour.

7. Testostérone pour utilisation selon la revendication 1 ou la revendication 2, où chaque dose comprend entre environ 5 mg et environ 15 mg de testostérone.

8. Testostérone pour utilisation selon la revendication 7, où chaque dose comprend environ 11 mg de testostérone.

9. Testostérone pour utilisation selon la revendication 1 ou la revendication 2, où la quantité totale de testostérone administrée au patient par jour est d'entre environ 10 mg et environ 120 mg.

10. Testostérone pour utilisation selon la revendication 9, où la quantité totale de testostérone administrée au patient par jour est d'entre environ 20 mg et environ 40 mg.

11. Testostérone pour utilisation selon la revendication 1 ou la revendication 2, ne comprenant pas l'administration d'un médicament quelconque sélectionné parmi le groupe composé de citrate de clomiphène, anastrozole et gonadotrophine chorionique humaine (HCG).
